# FASCICULE DE BREVET EUROPEEN

(11) **EP 0 831 949 B1**
(45) Date de publication et mention de la délivrance du brevet: **01.09.1999**
(21) Numéro de dépôt: 96922073.0
(22) Date de dépôt: 07.06.1996
(51) Int. Cl.: A61M 15/00, B05B 11/06

(54) **DISPOSITIF POUR FACILITER LE VIDAGE D'UNE CHAMBRE DE DOSAGE D'UN INHALATEUR**
VORRICHTUNG ZUM ERLEICHTERN DER ENTLEERUNG EINER INHALATOR-DOSIERKAMMER
DEVICE FOR ENHANCING THE EMPTYING OF AN INHALER METERING CHAMBER

(30) Priorité: 09.06.1995 FR 9506808
(43) Date de publication de la demande: 01.04.1998
(73) Titulaire: VALOIS S.A., 27110 Le Neubourg (FR)
(72) Inventeur: BRUNA, Pascal, F-76000 Rouen (FR); STRADELLA, Guiseppe, I-16032 Camogli (IT)
(74) Mandataire: CAPRI SARL
(86) Numéro de dépôt international: FR9600860
(87) Numéro de publication internationale: WO9641650

(56) Documents cités:
- WO-A-92/17233
- WO-A-93/18812
- WO-A-95/03846
- US-A- 1 732 566
- US-A- 3 522 659
- US-A- 5 394 868

## Description

La présente invention concerne un dispositif pour faciliter le vidage d'une chambre de dosage d'un distributeur de produit pulvérulent, en particulier d'un inhalateur de poudre.

L'utilisation d'inhalateur de poudre est aujourd'hui très répandue, notamment dans le domaine pharmaceutique, et les exigences sur les performances, l'efficacité et le coût de fabrication sont devenues très importantes.

Une des principales exigences est de garantir la meilleure reproductibilité possible de la dose de produit distribué à chaque actionnement de l'inhalateur. Ceci est particulièrement crucial lorsque l'inhalateur contient des médicaments dont le dosage doit être précis, ce qui est notamment le cas des médicaments contre l'asthme.

D'autre part, la qualité de la pulvérisation est également importante pour éviter que la dose de poudre expulsée ne comporte des agglomérats de poudre. De tels agglomérats empêchent en effet une bonne diffusion de la poudre dans les poumons et par conséquent diminuent l'efficacité du produit.

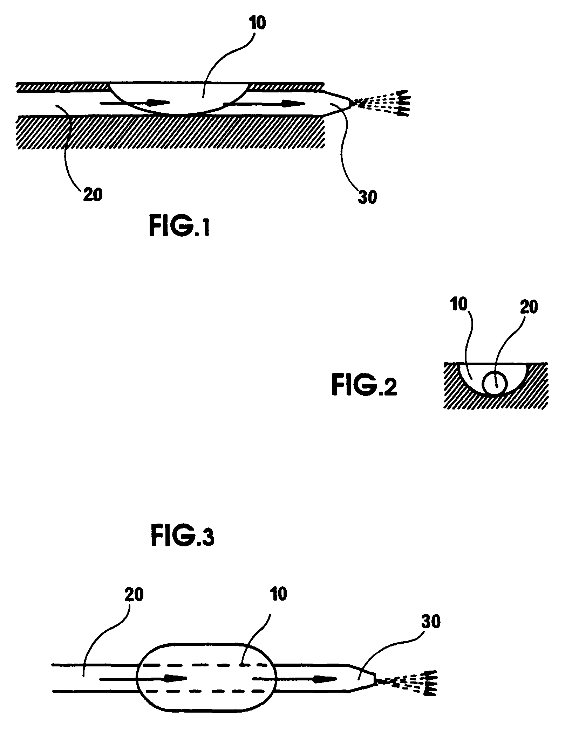

Les inhalateurs de poudre comprennent généralement une chambre de dosage remplie de poudre, ladite chambre de dosage étant vidée lors de l'actionnement de l'inhalateur, soit par un écoulement d'air comprimé, soit par un flux d'air généré par l'inhalation de l'utilisateur. Un exemple d'un tel appareil connu, qui est divulgué notamment dans le document WO 93/18812, est représenté partiellement sur les figures 1 à 3. Les figures 1 à 3 montrent respectivement en coupe latérale, transversale et horizontale une chambre de dosage 10, traversée par un canal d'expulsion 20 qui s'étend jusqu'à une embouchure 30 du distributeur. La chambre de dosage 10 est remplie de produit à partir d'un réservoir de produit (non représenté) et, lors de l'actionnement de l'appareil, un écoulement d'air représenté schématiquement par les flèches sur les figures 1 et 3, passe à travers ledit canal d'expulsion 20 pour vider la chambre de dosage 10 et amener la dose de produit jusqu'à l'embouchure 30 du distributeur, où elle est distribuée à l'utilisateur.

Pour obtenir une bonne reproductibilité de la dose, il est nécessaire d'une part de remplir complètement la chambre de dosage après chaque utilisation, et d'autre part, de la vider complètement lors de l'actionnement de l'appareil. En effet, si la chambre de dosage n'est pas complètement vidée, le volume de la dose expulsée ne correspond plus à la dose prescrite et la poudre restant dans la chambre de dosage peut former des zones d'accumulation de poudre qui empêchent une bonne reproductibilité de la dose.

L'expulsion de la dose de poudre, c'est-à-dire le vidage de la chambre de dosage, dans un tel appareil est une opération très difficile à réaliser et dépend de nombreux paramètres parmi lesquels :
- la forme et la dimension de la chambre de dosage, et donc la quantité de médicament à distribuer,
- la forme du conduit d'expulsion, qui souvent ne correspond pas à la forme de la chambre de dosage. Ainsi, comme visible en particulier sur la figure 2, la chambre de dosage 10 a généralement une section transversale supérieure à celle du canal d'expulsion 20.
- la dimension du conduit d'expulsion, devant garantir une vitesse d'air appropriée.
- les conditions d'écoulement de l'air, dépendant de la source d'air et de la dimension du conduit d'expulsion.

Dans des dispositifs connus, représentés sur les figures 1 à 3, le conduit d'expulsion 20 est généralement cylindrique et l'écoulement d'air se conforme à la forme du conduit. Ainsi, lorsque l'écoulement d'air pénètre dans la chambre de dosage 10 remplie de poudre, il suit une trajectoire sensiblement rectiligne entre l'entrée et la sortie de ladite chambre de dosage. Dans ce cas, en particulier lorsque la chambre de dosage a un volume important, l'écoulement d'air a tendance à d'abord "creuser un tunnel" dans la poudre, c'est-à-dire à former un passage représenté en pointillé sur la figure 3, s'étendant directement entre l'entrée et la sortie de la chambre de dosage 10, ledit passage correspondant sensiblement à un prolongement du canal d'expulsion 20 dans la chambre de dosage. Ainsi, une partie seulement de la poudre contenue dans la chambre de dosage est expulsée. Ensuite, dans une seconde étape, si la chambre de dosage n'est pas trop grande, l'écoulement d'air entraîne la poudre qui est située autour dudit "tunnel".

Bien que cet arrangement se soit avéré efficace pour des chambres de dosage relativement petites, il présente des inconvénients importants dans le cas de doses importantes à délivrer. Dans ce cas, en effet, une partie de la poudre, disposée dans des zones de bords de la chambre de dosage c'est-à-dire au voisinage de ses parois, n'est pas expulsée et s'accumule dans ladite chambre, ce qui engendre des problèmes de reproductibilité de la dose.

Une augmentation du volume de la chambre de dosage dans un tel inhalateur connu nécessite donc une adaptation correspondante du canal d'expulsion et donc de la source de l'écoulement d'air, ce qui augmente considérablement les coûts de fabrication d'un tel appareil.

Dans un autre dispositif connu, divulgué dans le document WO 95/28980, la chambre de dosage est partiellement délimitée par un insert faisant saillie dans le canal d'expulsion. Cette chambre de dosage comporte également des zones de bord difficile à vider. D'autre part, de par la présence de l'insert qui fait saillie dans le canal à travers une paroi de celui-ci, des problèmes de rétention de poudre peuvent se produire sur la partie aval de l'insert, de sorte que la reproductibilité de la dose n'est pas garantie.

L'invention a pour but de fournir un dispositif pour faciliter le vidage d'une chambre de dosage afin de garantir une parfaite reproductibilité de la dose.

L'invention a aussi pour but de fournir un dispositif pour faciliter le vidage d'une chambre de dosage, dans lequel la dose de poudre est complètement désagglomérée lors de son expulsion.

Les documents US-1,732,566 et US-3,522,659 divulguent des dispositifs comportant des moyens d'écoulement pour modifier la configuration de l'écoulement d'air dans la chambre de dosage pour en faciliter le vidage. Ces moyens sont disposés dans la chambre de dosage, de sorte qu'une modification des dimensions de la chambre de dosage entraîne une modification desdits moyens d'écoulement.

L'invention a donc également pour but de fournir un dispositif pour faciliter le vidage d'une chambre de dosage, permettant de modifier le volume de la chambre de dosage sans devoir modifier une autre partie du distributeur de produit.

L'invention a encore pour but de fournir un dispositif pour faciliter le vidage d'une chambre de dosage, permettant de garantir une parfaite reproductibilité de la dose, indépendamment du volume de la chambre de dosage, sans augmenter les coûts de fabrication du distributeur de produit.

L'invention a donc pour objet un dispositif pour faciliter le vidage d'une chambre de dosage d'un distributeur de produit pulvérulent, ledit distributeur comportant un canal d'expulsion traversant ladite chambre de dosage et des moyens pour fournir un écoulement d'air destine, lors de l'actionnement du distributeur, à passer dans ledit canal d'expulsion pour vider ladite chambre de dosage, ledit dispositif comportant des moyens d'écoulement adaptés à configurer l'écoulement d'air de telle sorte qu'il vide totalement ladite chambre de dosage, caractérisé en ce que lesdits moyens d'écoulement sont disposés dans le canal d'expulsion, à l'extérieur et en amont de ladite chambre de dosage dans le sens d'écoulement dudit écoulement d'air.

En particulier, ladite chambre de dosage comporte au moins une partie de section transversale supérieure à la section transversale dudit canal d'expulsion formant au moins une zone de bord. lesdits moyens d'écoulement étant adaptés à diriger au moins une partie de l'écoulement d'air dans ladite au moins une zone de bord de ladite chambre de dosage, de telle manière à vider totalement ladite chambre de dosage, y compris dans ladite au moins une zone de bord.

L'invention est donc adaptée à modifier la configuration de l'écoulement d'air lorsque celui-ci pénètre dans la chambre de dosage et notamment de telle manière qu'au moins une partie de l'écoulement d'air soit dirigée vers lesdites zones de bord pour expulser la totalité de la dose, indépendamment de la relation entre la section transversale de la chambre de dosage et celle du canal d'expulsion. Il est ainsi possible de fabriquer des inhalateurs avec des chambres de dosages de volume et de forme différents sans avoir besoin de modifier une autre partie de l'inhalateur, ce qui est un avantage économique important. D'autre part, on garantit une reproductibilité parfaite de la dose pour ces différents inhalateurs.

Selon un mode de réalisation préféré de l'invention, lesdits moyens d'écoulement comportent un insert disposé fixement dans ledit canal d'expulsion. Cet insert étant indépendant du volume de la chambre de dosage, il garantit donc le vidage de celle-ci, quel que soit son volume. En particulier, une modification du volume de la chambre de dosage ne nécessite pas une modification de l'insert.

Selon une première variante, ledit insert comporte un noyau central plein, l'écoulement d'air s'écoulant le long de la surface extérieure dudit noyau central.

Selon une seconde variante, ledit insert comporte un noyau central comportant une ouverture axiale de long de son axe central longitudinal, une partie de l'écoulement d'air s'écoulant à travers ladite ouverture axiale et le restant le long de la surface extérieure dudit noyau central.

Ainsi, l'insert permet de maintenir la même vitesse de l'écoulement d'air tout en augmentant la surface de contact entre l'écoulement d'air et la poudre contenue dans la chambre de dosage.

De préférence, ledit insert est dimensionné de telle sorte qu'il est maintenu fixement par coincement dans le canal d'expulsion. Lors de la fabrication d'un inhalateur, il suffit donc d'emmancher un insert selon l'invention, en général une petite pièce en plastique ou en métal très peu coûteuse, dans le canal d'expulsion, en amont de la chambre de dosage. Aucune étape de fixation supplémentaire de l'insert dans le canal, qui augmenterait les coûts de fabrication, n'est nécessaire.

Selon un mode de réalisation particulier de l'invention, le canal d'expulsion a une section transversale circulaire ou oblongue et ledit insert a une section transversale polygonale.

Selon un autre mode de réalisation particulier de l'invention, le canal d'expulsion a une section polygonale et ledit insert a une section transversale circulaire ou oblongue.

Selon une forme de réalisation préférée de l'invention, ledit insert comporte en outre au moins une nervure s'étendant environ axialement le long de la surface extérieure dudit noyau central.

Avantageusement, le canal d'expulsion a une section transversale sensiblement circulaire, le noyau de l'insert ayant une section transversale sensiblement circulaire inférieure à celle dudit canal d'expulsion, ledit insert comportant plusieurs nervures s'étendant environ axialement le long de la surface extérieure dudit noyau central, le diamètre extérieur de l'insert au niveau desdites nervures étant environ identiques au diamètre intérieur dudit canal d'expulsion, de sorte que l'insert est maintenu dans ledit canal par coincement au niveau desdites nervures.

Avantageusement, l'insert comporte au moins trois nervures qui sont réparties régulièrement autour du noyau central, de sorte que ledit noyau central est centré au milieu dudit canal d'expulsion.

De préférence, l'insert comporte quatre nervures identiques diamétralement opposées deux à deux autour dudit noyau central.

Selon une première variante, ladite au moins une nervure s'étend axialement sur le noyau central parallèlement à l'axe longitudinal du canal d'expulsion.

Selon une seconde variante, ladite au moins une nervure s'étend axialement sur le noyau central de manière vrillée par rapport à l'axe longitudinal du canal d'expulsion, de sorte que l'insert crée un écoulement d'air tourbillonnant.

De préférence, la section transversale de l'extrémité aval dudit noyau central de l'insert, dans le sens d'écoulement de l'écoulement d'air, est supérieure à la section transversale de l'extrémité amont dudit noyau central, de sorte que l'insert crée un écoulement d'air pénétrant dans la chambre de dosage selon une trajectoire conique.

Eventuellement, lesdits moyens d'écoulement comportent au moins une rainure ou nervure s'étendant hélicoïdalement sur la paroi intérieure du canal d'expulsion. Dans cette variante, on peut éventuellement se dispenser de l'insert, le profil hélicoidal formé dans le canal d'expulsion créant un écoulement d'air tourbillonnant dans la chambre de dosage.

Selon encore un autre mode de réalisation avantageuse, ladite chambre de dosage est délimitée partiellement par un élément de dosage disposé fixement dans le canal d'expulsion, ledit élément de dosage étant disposé en aval desdits moyens d'écoulement dans le sens d'écoulement dudit écoulement d'air.

De préférence, ledit élément de dosage est disposé en éloignement des parois du canal d'expulsion, de telle sorte que l'écoulement d'air s'écoule sur toute la périphérie dudit élément de dosage. Ainsi, aucune rétention de poudre ne peut se produire sur une quelconque partie dudit élément de dosage et la totalité de la dose sera expulsée à chaque actionnement.

Avantageusement, ledit élément de dosage se termine en pointe à son extrémité aval dans le sens de l'écoulement de l'air.

De préférence, ledit élément de dosage est fixé auxdits moyens d'écoulement.

Avantageusement, lesdits moyens d'écoulement sont réalisés sous la forme d'un insert fixé à un organe de fixation, ledit organe de fixation étant fixé dans un prolongement axial du canal d'expulsion de telle sorte que l'insert fait saille dans ledit canal d'expulsion en éloignement des parois dudit canal, l'écoulement d'air s'écoulant sur toute la périphérie dudit insert. Cet insert peut avoir l'une quelconque des formes décrites précédemment, et en particulier, ledit insert à une section transversale rectangulaire et ledit élément de dosage a une section transversale circulaire. Ainsi, la configuration de l'écoulement d'air est modifiée par cet insert, et cet écoulement d'air modifié, comportant des turbulences en raison de la forme de l'insert, vient ensuite vider complètement la chambre de dosage. Le fait que l'écoulement d'air s'écoule tout autour de la périphérie de l'élément de dosage et que ce dernier se termine en pointe garantit une parfaite pulvérisation de la totalité de la dose.

De préférence, ledit élément de dosage, ledit insert et ledit organe de fixation sont réalisés en une seule pièce. Cette mise en oeuvre facilite la mise en place du dispositif insert + élément de dosage, et diminue donc ses coûts de fabrication et de montage. De plus, pour modifier la chambre de dosage, il suffit de modifier l'élément de dosage, sans modifier une quelconque autre pièce de l'appareil. ce qui limite également les coûts.

Avantageusement, ledit élément de dosage comporte une zone, telle qu'une coupelle, définissant le volume de la chambre de dosage. Ainsi, pour modifier le volume de la chambre de dosage, il suffit de modifier cette zone de l'élément de dosage, ce qui est très simple et peu coûteux.

Avantageusement, ledit écoulement d'air est un écoulement d'air comprimé crée dans le distributeur par un dispositif de pompe précontrainte déclenchable lors de l'actionnement du distributeur.

D'autres caractéristiques et avantages apparaîtront au cours de la description détaillée suivante donnée à titre d'exemple non limitatif, en regard des dessins joints, sur lesquels :
- la figure 1 est une vue schématique en coupe latérale d'une chambre de dosage et d'un canal d'expulsion d'un inhalateur de l'art antérieur,
- la figure 2 est une vue en coupe transversale de l'arrangement de la figure 1,
- la figure 3 est une vue en coupe horizontale de l'arrangement de la figure 1,
- la figure 4 est une vue similaire à celle de la figure 1, incorporant un insert selon un premier mode de réalisation de l'invention,
- la figure 5 est une vue similaire à celle de la figure 2, incorporant l'insert de la figure 4,
- la figure 6a représente une vue schématique en coupe latérale d'un insert selon un mode de réalisation avantageux de l'invention,
- la figure 6b représente une vue schématique en coupe transversale de l'extrémité amont de l'insert de la figure 6a,
- la figure 6c représente une vue schématique en coupe transversale de l'extrémité aval de l'insert de la figure 6a,
- la figure 7 est une vue similaire à celle de la figure 5, incorporant un insert selon une variante de réalisation de l'invention,
- la figure 8 est une vue similaire à celle de la figure 7 incorporant une autre variante de l'insert selon l'invention,
- la figure 9 est une vue schématique en perspective d'un insert selon un autre mode de réalisation avantageux de l'invention,
- la figure 10 est une vue schématique d'une partie d'un distributeur de produit incorporant l'insert de la figure 9,
- la figure 11 est une vue similaire à celle de la figure 10, incorporant un dispositif selon un autre mode de réalisation de l'invention, et
- les figures 12a et 12b représentent des vues schématiques en coupe transversale, respectivement à travers l'insert et l'élément de dosage du dispositif de la figure 11.

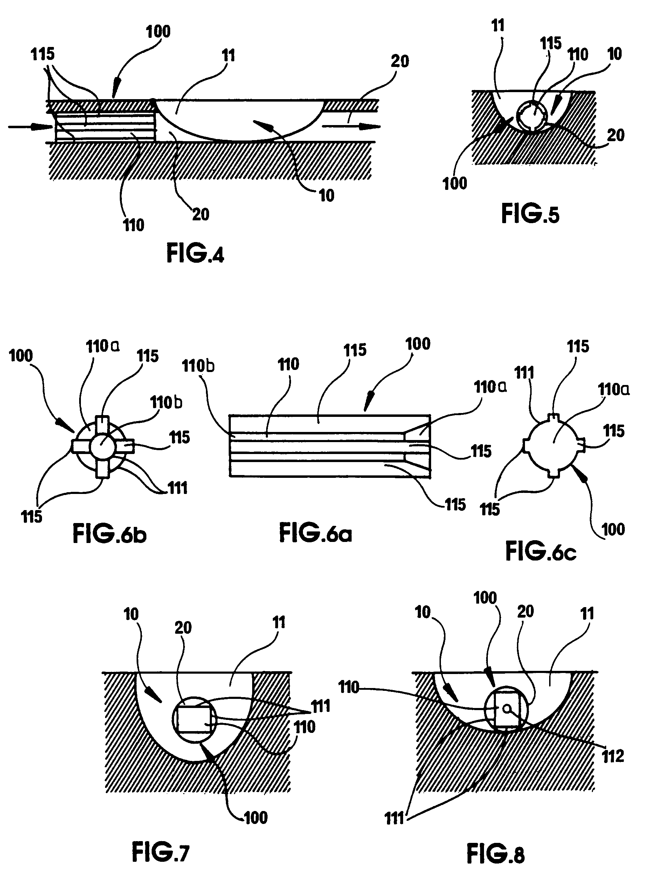

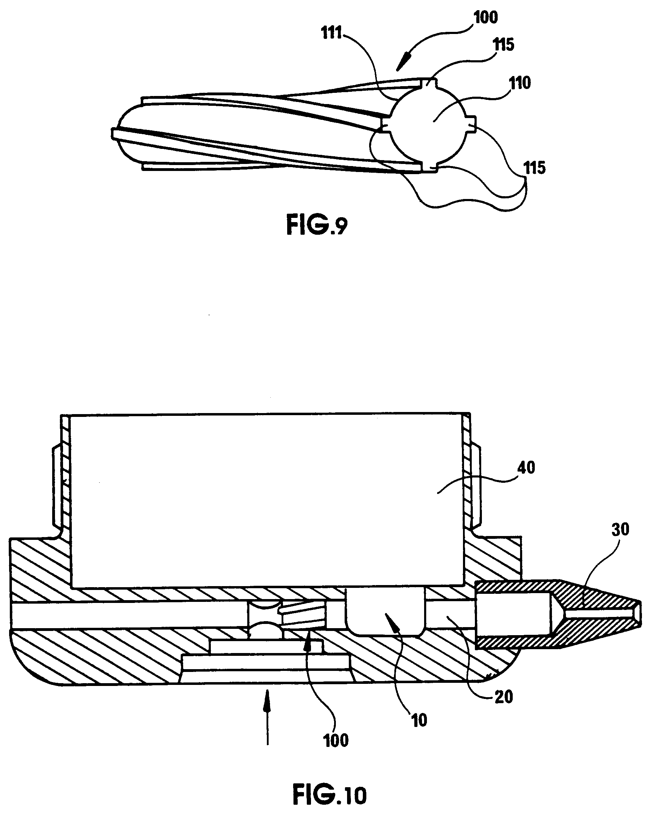

En référence à la figure 10, un distributeur de produit tel qu'un inhalateur de poudre comprend une chambre de dosage 10 destinée à être remplie de produit à partir d'un réservoir 40 au moyen d'un mécanisme de remplissage (non représenté). La chambre de dosage 10 est traversée par un canal d'expulsion 20 qui s'étend d'une part en direction d'une embouchure de distribution 30 de l'inhalateur, et d'autre part en direction d'une source de écoulement d'air (non représentée). Comme visible également sur la figure 4, l'invention prévoit de disposer dans le canal d'expulsion 20, en amont de la chambre de dosage 10 dans le sens d'écoulement de l'écoulement d'air représenté schématiquement par les flèches, des moyens d'écoulement, tel qu'un insert 100, destinés à améliorer le vidage de la chambre de dosage lors de l'actionnement du distributeur. Cet insert 100, qui est fixé de préférence par coincement dans ledit canal d'expulsion 20, est destiné à modifier la configuration de l'écoulement d'air, et notamment sa forme, lorsque celui-ci pénètre dans la chambre de dosage 10 de telle manière que ledit écoulement d'air soit dirigé vers des zones de bord 11 de la chambre de dosage 10, qui sont disposées à l'extérieur de la partie du conduit d'expulsion 20 qui traverse ladite chambre de dosage 10. Comme visible en particulier sur les figures 4, 5, 7 et 8, la section transversale la chambre de dosage 10 est généralement supérieure à la section transversale du canal d'expulsion 20, lesdites zones de bord 11 étant par conséquent les parties de la chambre de dosage 10 situées à proximité de ses parois. L'insert 100 permet également de créer des turbulences ou des tourbillonnements dans l'écoulement d'air de sorte que lorsqu'elles pénètrent dans la chambre de dosage, ces turbulences ou tourbillonnements agissent pour désagglomérer la dose de poudre, si nécessaire. Ainsi, la totalité de la dose est expulsée de manière optimale.

Selon l'invention, l'insert 100 comporte un noyau central 110 qui s'étend de préférence jusqu'à l'entrée de ladite chambre de dosage 10. Selon un mode de réalisation préfère de l'invention, le canal d'expulsion et le noyau central 110 de l'insert 100 ont chacun une section transversale sensiblement circulaire, la section transversale du noyau central étant évidement légèrement inférieure à la section transversale dudit canal d'expulsion 20. L'insert 100 a donc une forme générale environ cylindrique. De préférence, l'insert 100 comporte en outre au moins une nervure 115 qui s'étend environ axialement le long de la surface extérieure 111 dudit noyau central 110 de l'insert 100. Avantageusement, l'insert comporte au moins trois nervures 115 réparties autour dudit noyau central 110, lesdites nervures étant réalisées de telle sorte que le diamètre extérieur de l'insert 100, au niveau des nervures 115 est environ identique ou très légèrement supérieure au diamètre intérieur dudit canal d'expulsion 20, ce qui permet de maintenir fixement l'insert 100 dans le canal 20 par coincement au niveau desdites nervures 115. En référence aux figures 4 et 5, l'insert 100 comporte quatre nervures identiques 115 s'étendant parallèlement à l'axe longitudinal de l'insert. Ces nervures 115 sont disposées diamétralement opposées deux à deux autour dudit noyau central 110 de sorte que ledit noyau central 110 est centré au milieu dudit canal d'expulsion 20. Dans cet exemple, le noyau central 110 est plein de sorte que l'écoulement d'air issu de la source de écoulement d'air s'écoule, au niveau de l'insert 100, sur la surface extérieure 111 du noyau central 110 entre les différentes nervures 115. Ainsi, la forme de l'écoulement d'air qui, avant de venir au contact de l'insert 100, est sensiblement en forme de cylindre, ce qui correspond à la forme du canal d'expulsion 20, est modifié par l'insert 100 de telle sorte que lorsque l'écoulement d'air débouche dans la chambre de dosage 10, il a sensiblement la forme d'un tube creux de sorte que la partie active de l'écoulement d'air est plus proche des parois de la chambre de dosage 10 et n'est pas dirigé directement vers la sortie de ladite chambre de dosage 10.

Selon une variante avantageuse de réalisation, représentée sur les figures 6a, 6b et 6c, l'insert 100 de forme générale cylindrique comporte un noyau central 110 dont la section transversale au niveau de son extrémité amont 110b, dans le sens d'écoulement de l'écoulement d'air, est inférieure à la section transversale au niveau de son extrémité aval 110a. Ainsi, l'écoulement d'air qui s'écoule le long de la surface extérieure 111 du noyau central 110 de l'insert 100 pénètre dans la chambre de dosage 10 avec une forme de écoulement d'air environ tronconique s'évasant vers l'extérieur de sorte que ledit écoulement d'air est encore davantage dirigé vers les zones de bord 11 de la chambre de dosage 10 lorsqu'il pénètre dans celle-ci.

Une autre variante de réalisation avantageuse est représentée sur la figure 9. L'insert 100, toujours de forme générale cylindrique, comporte comme précédemment un noyau central 110 et quatre nervures 115 s'étendant le long de sa surface extérieure 111. Dans cette variante, lesdites nervures 115 s'étendent sur ladite surface extérieure 111 du noyau central 110 de manière vrillée par rapport à l'axe longitudinal du noyau central 110 de l'insert, qui correspond à l'axe longitudinal central du canal d'expulsion 20. Ainsi, l'écoulement d'air qui s'écoule le long de ladite surface extérieure 111 du noyau central 110, entre lesdites nervures 115, pénètre dans la chambre de dosage en tourbillonnant ce qui favorise grandement le vidage de celle-ci. C'est cette variante de l'insert 100 qui est représentée schématiquement dans la vue d'ensemble de la figure 10.

En référence aux figures 7 et 8, d'autres variantes dudit insert 100 sont représentées schématiquement. Ainsi, ledit noyau central 110 peut ne pas comporter de nervures 115. Dans ce cas, si le canal d'expulsion 20 a une section transversale circulaire, la section transversale de l'insert peut être polygonale, de préférence carrée ou rectangle, de telle sorte qu'il puisse être coincé dans le canal d'expulsion 20 au niveau de ses angles. Comme précédemment, l'écoulement d'air s'écoule le long de la surface extérieure 111 dudit noyau central 110. Comme représenté sur la figure 8, ledit noyau 110 peut en outre comporter une ouverture axiale 112 s'étendant le long de son axe central longitudinal, de sorte que l'écoulement d'air qui arrive au niveau dudit insert 100 se sépare en une partie qui s'écoule axialement à travers ladite ouverture axiale 112 et une partie qui s'écoule le long de la surface extérieure 111 dudit noyau central 110. Lorsque, comme représenté sur les figures 7 et 8, le canal d'expulsion 20 est de section transversale circulaire et l'insert 100 a un noyau central de section transversale rectangulaire, on peut modifier la répartition de l'écoulement d'air sur les quatre faces dudit noyau central de forme générale parallélépipédique, en fonction de la forme de la chambre de dosage 10. Ainsi, lorsque la chambre de dosage 10 est plus profonde que large (figure 7), il est avantageux que les plus grands côtés de l'insert 100 soient orientés horizontalement, pour dévier une plus grande partie de l'écoulement d'air vers les parois supérieures et inférieures de la chambre de dosage 10. Au contraire, lorsque la chambre de dosage est plus large que profonde (figure 8), les plus grands côtés du parallélépipède 110 sont avantageusement disposés de manière verticale, pour dévier une plus grande partie de l'écoulement d'air vers les parois latérales de la chambre de dosage 10. Bien entendu, toute autre forme polygonale de la section transversale de l'insert 100 est imaginable pour obtenir le résultat souhaité, à savoir un vidage complet de la chambre de dosage 10 par l'écoulement d'air émis lors de l'actionnement de l'appareil d'inhalation.

Las différents modes de réalisation de l'insert ont été décrits en relation à un canal d'expulsion dont la section transversale est environ circulaire, mais ils s'adaptent également à un canal d'expulsion de section transversale elliptique ou oblong. De même, un canal d'expulsion dont la section transversale serait polygonale (en particulier rectangulaire ou carrée), peut recevoir un insert selon un des modes de réalisation de l'invention décrit précédemment. D'autre part, si on se réfère aux modes de réalisation représentés sur les figures 7 et 8, les formes des sections transversales respectives du canal 20 et de l'insert 100 peuvent être inversées, c'est-à-dire que le canal d'expulsion 20 peut avoir une section transversale rectangulaire, auquel cas l'insert 100 aura avantageusement un noyau central 110 de section transversale circulaire ou elliptique.

Eventuellement, on peut prévoir une ou plusieurs rainures ou nervures s'étendant hélicoïdalement sur la surface intérieure du canal d'expulsion. Dans ce cas, une partie de l'écoulement d'air s'écoule le long de ces rainures ou nervures de sorte qu'il est créé un écoulement d'air tourbillonnant dans la chambre de dosage. Ainsi, on peut se passer de l'insert.

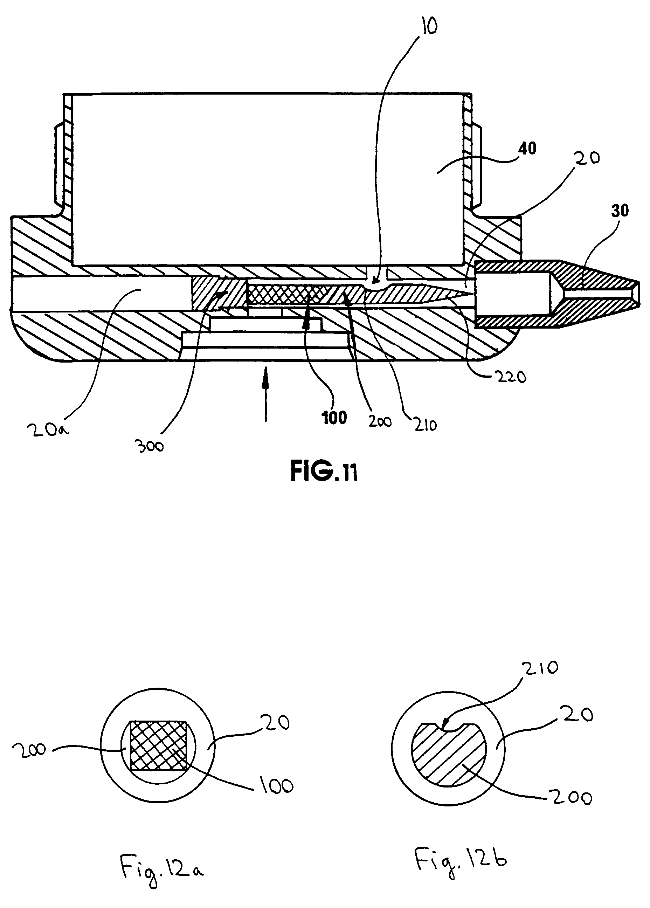

Selon un autre mode de réalisation préféré de l'invention, représenté sur la figures 11, 12a et 12b, la chambre de dosage 10 est partiellement délimitée par un élément de dosage 200, en particulier au moyen d'une zone définissant le volume de ladite chambre de dosage. Cette zone 210 peut être réalisée sous la forme d'une coupelle, comme représentée sur les figures 11 et 12b, mais pourrait aussi être formé d'une quelconque autre manière, par exemple sous la forme d'un simple plat supportant la dose de poudre alimentée à partir du réservoir 40. Selon l'invention, ledit élément de dosage 200 est disposé fixement dans le canal d'expulsion 20, en aval dudit insert 100. De préférence, l'élément de dosage 200 est fixé dans le canal d'expulsion 20 en éloignement des parois dudit canal de telle sorte que l'écoulement d'air s'écoule sur toute sa périphérie. Ainsi, l'écoulement d'air expulse la totalité de la poudre puisqu'aucune rétention de poudre ne peut se produire contre une partie dudit élément de dosage 200. Pour encore davantage améliorer ceci, l'extrémité aval (220) de l'élément de dosage 200 se termine avantageusement en pointe.

De préférence, comme représenté sur la figure 11, l'élément de dosage 200 est fixé à l'insert 100, qui est lui même fixé avantageusement à un organe de fixation 300. Cet organe de fixation 300 est fixé dans un prolongement axial 20b du canal d'expulsion 20, par exemple par coincement ou par ancrage. Ainsi, l'insert 100 et l'élément de dosage 200 font saillie axialement dans le canal d'expulsion 20. De préférence, l'insert 100 est également disposé en éloignement des parois du canal 20, de sorte que l'écoulement d'air s'écoule autour de toute la périphérie de l'insert 100. Selon un mode de réalisation avantageuse de l'invention, l'organe de fixation 300, l'insert 100 et l'élément de dosage 200 sont réalisés en une seule pièce, de préférence en matériau plastique. Ainsi la fabrication et le montage du dispositif de l'invention sont très simples et donc peu coûteux, et une modification de la chambre de dosage, par exemple de son volume, peut être réalisée très facilement en ne modifiant que ledit élément de dosage 200.

De préférence, comme représenté sur les figures 12a et 12b, la section transversale de l'insert 100 est sensiblement rectangulaire et celle de l'élément de dosage 200 est sensiblement circulaire. Ainsi, l'insert 100 modifie la configuration de l'écoulement d'air et la transition entre l'insert et l'élément de dosage 200 favorise encore la création de turbulences, de sorte que l'expulsion totale de la dose de poudre sous forme finement pulvérisée est garantie.

## Revendications

1. Dispositif pour faciliter le vidage d'une chambre de dosage (10) d'un distributeur de produit pulvérulent ledit distributeur comportant un canal d'expulsion (20) traversant ladite chambre de dosage (10) et des moyens pour fournir un écoulement d'air destiné, lors de l'actionnement du distributeur, à passer dans ledit canal d'expulsion (20) pour vider ladite chambre de dosage (10), ledit dispositif comportant des moyens d'écoulement (100) adaptés à configurer l'écoulement d'air de telle sorte qu'il vide totalement ladite chambre de dosage (10), caractérisé en ce que lesdits moyens d'écoulement (100) sont disposés dans le canal d'expulsion (20), a' l'extérieur et en amont de ladite chambre de dosage (10) dans le sens d'écoulement dudit écoulement d'air.

2. Dispositif selon la revendication 1, dans lequel ladite chambre de dosage (10) comporte au moins une partie de section transversale supérieure à la section transversale dudit canal d'expulsion (20) formant au moins une zone de bord (11), lesdits moyens d'écoulement (100) étant adaptés à diriger au moins une partie de l'écoulement d'air dans ladite au moins une zone de bord (11) de ladite chambre de dosage (10), de telle manière à vider totalement ladite chambre de dosage (10), y compris dans ladite au moins une zone de bord (11).

3. Dispositif selon la revendication 1 ou 2, dans lequel lesdits moyens d'écoulement (100) comportent un insert (100) disposé fixement dans ledit canal d'expulsion.

4. Dispositif selon la revendication 3, dans lequel ledit insert (100) comporte un noyau central (110) plein, l'écoulement d'air s'écoulant le long de la surface extérieure (111) dudit noyau central (110).

5. Dispositif selon la revendication 3, dans lequel ledit insert (100) comporte un noyau central (110) comportant une ouverture axiale (112) le long de son axe central longitudinal, une partie de l'écoulement d'air s'écoulant à travers ladite ouverture axiale (112) et le restant le long de la surface extérieure (111) dudit noyau central (110).

6. Dispositif selon l'une quelconque des revendications 3 ou 5, dans lequel ledit insert (100) est dimensionné de telle sorte qu'il est maintenu fixement par coincement dans le canal d'expulsion (20).

7. Dispositif selon l'une quelconque des revendications 3 à 6, dans lequel le canal d'expulsion (20) a une section transversale circulaire ou oblongue et ledit insert (100) a une section transversale polygonale.

8. Dispositif selon l'une quelconque des revendications 3 à 6, dans lequel le canal d'expulsion (20) a une section polygonale et ledit insert (100) a une section transversale circulaire ou oblongue.

9. Dispositif selon l'une quelconque des revendications 3 à 8, dans lequel ledit insert (100) comporte en outre au moins une nervure (115) s'étendant environ axialement le long de la surface extérieure (111) dudit noyau central (110).

10. Dispositif selon la revendication 9, dans lequel le canal d'expulsion (20) a une section transversale sensiblement circulaire, le noyau (110) de l'insert (100) ayant une section transversale sensiblement circulaire, inférieure à celle dudit canal d'expulsion (20), ledit insert (100) comportant plusieurs nervures (115) s'étendant environ axialement le long de la surface extérieure (111) dudit noyau central (110), le diamètre extérieur de l'insert (100) au niveau desdites nervures (115) étant environ identique au diamètre intérieur dudit canal d'expulsion (20), de sorte que l'insert (100) est maintenu dans ledit canal (20) par coincement au niveau desdites nervures (115).

11. Dispositif selon la revendication 9 ou 10, dans lequel l'insert (100) comporte au moins trois nervures (115) qui sont réparties régulièrement autour du noyau central (110), de sorte que ledit noyau central (110) est centré au milieu dudit canal d'expulsion (20).

12. Dispositif selon l'une quelconque des revendications 9 à 11, dans lequel l'insert (100) comporte quatre nervures (115) identiques diamétralement opposés deux à deux autour dudit noyau central (110).

13. Dispositif selon l'une quelconque des revendications 9 à 12, dans lequel ladite au moins une nervure (115) s'étend axialement sur le noyau central (110) parallelement à l'axe longitudinal du canal d'expulsion (20).

14. Dispositif selon l'une quelconque des revendications 9 à 12, dans lequel ladite au moins une nervure (115) s'étend axialement sur le noyau central (110) de manière vrillée par rapport à l'axe longitudinal du canal d'expulsion (20), de sorte que l'insert (100) crée un écoulement d'air tourbillonnant dans la chambre de dosage (10).

15. Dispositif selon l'une quelconque des revendications 3 à 14, dans lequel la section transversale de l'extrémité aval (110a) dudit noyau central (110) de l'insert (100), dans le sens d'écoulement de l'écoulement d'air, est supérieure à la section transversale de l'extrémité amont (110b) dudit noyau central (110), de sorte que l'insert (100) crée un écoulement d'air pénétrant dans la chambre de dosage (10) selon une trajectoire conique.

16. Dispositif selon l'une quelconque des revendications précédentes, dans lequel lesdits moyens d'écoulement (100) comportent au moins une rainure ou nervure s étendant hélicoïdalement sur la paroi intérieure du canal d'expulsion (20).

17. Dispositif selon l'une quelconque des revendications précédentes, dans lequel ladite chambre de dosage (10) est délimitée partiellement par un élément de dosage (200) disposé fixement dans le canal d'expulsion (20), ledit élément de dosage (200) étant disposé en aval desdits moyens d'écoulement (100) dans le sens d'écoulement dudit écoulement d'air.

18. Dispositif selon la revendication 17, dans lequel ledit élément de dosage (200) est disposé en éloignement des parois du canal d'expulsion (20), de telle sorte que l'écoulement d'air s'écoule sur toute la périphérie dudit élément de dosage (200).

19. Dispositif selon la revendication 17 ou 18, dans lequel ledit élément de dosage (200) se termine en pointe (220) à son extrémité aval dans le sens d'écoulement de l'air.

20. Dispositif selon l'une quelconque des revendications 17 à 19, dans lequel ledit élément de dosage (200) est fixé auxdits moyens d'écoulement (100).

21. Dispositif selon la revendication 20, dans lequel lesdits moyens d'écoulement (100) sont réalisés sous la forme d'un insert (100) fixé à un organe de fixation (300), ledit organe de fixation (300) étant fixé dans un prolongement axial (20a) du canal d'expulsion (20) de telle sorte que l'insert (100) fait saillie dans ledit canal d'expulsion (20) en éloignement des parois dudit canal (20), l'écoulement d'air s'écoulant sur toute la périphérie dudit insert (100).

22. Dispositif selon la revendication 21, dans lequel ledit insert (100) a une section transversale rectangulaire et ledit élément de dosage (200) a une section transversale circulaire.

23. Dispositif selon la revendication 21 ou 22, dans lequel ledit élément de dosage (200), ledit insert (100) et ledit organe de fixation (300) sont réalisés en une seule pièce.

24. Dispositif selon l'une quelconque des revendications 17 à 23, dans lequel ledit élément de dosage (200) comporte une zone (210), telle qu'une coupelle, définissant le volume de la chambre de dosage (10).

25. Dispositif selon l'une quelconque des revendications précédentes, dans lequel ledit écoulement d'air est un écoulement d'air comprimé crée dans le distributeur par un dispositif de pompe précontrainte déclenchable lors de l'actionnement du distributeur.

## Claims

1. A device for making it easier to empty a metering chamber (10) of a powder dispenser, said dispenser including an expulsion channel (20) passing through said metering chamber (10), and means for delivering an air flow which is intended, when the dispenser is actuated, to pass along said expulsion channel (20) to empty said metering chamber (10), said device including flow means (100) adapted to configure the air flow in such a manner that it empties said metering chamber (10) completely, the device being characterized in that said flow means (100) are disposed in the expulsion channel (20) outside and upstream from said metering chamber (10) in the flow direction of said air flow.

2. A device according to claim 1, in which said metering chamber (10) includes at least one portion of cross-sectional area greater than the cross-sectional area of said expulsion channel (20), forming at least one side zone (11), said flow means (100) being adapted to direct at least a portion of the air flow into said at least one side zone (11) of said metering chamber (10) in such a manner as to empty said metering chamber (10) completely, including in said at least one side zone (11).

3. A device according to claim 1 or 2, in which said flow means (100) include an insert (100) disposed in fixed manner in said expulsion channel.

4. A device according to claim 3, in which said insert (100) includes a solid central core (110), the air flow flowing over the outside surface (111) of said central core (110).

5. A device according to claim 3, in which said insert (100) includes a central core (110) having an axial opening (112) along its longitudinal central axis, a portion of the air flow flowing through said axial orifice (112) and the remainder flows along the outside surface (111) of said central core (110).

6. A device according to any one of claims 3 to 5, in which said insert (100) is dimensioned in such a manner as to be held in fixed manner by being jammed in the expulsion channel (20).

7. A device according to any one of claims 3 to 6, in which the expulsion channel (20) is of circular or oblong cross-section, and said insert (100) is of polygonal cross-section.

8. A device according to any one of claims 3 to 6, in which the expulsion channel (20) is polygonal in section and said insert (100) is of circular or oblong cross-section.

9. A device according to any one of claims 3 to 8, in which said insert (100) further includes at least one rib (115) extending substantially axially along the outside surface (111) of said central core (110).

10. A device according to claim 9, in which the expulsion channel (20) is of substantially circular cross-section, the core (110) of the insert (100) being of substantially circular cross-section, smaller than that of said expulsion channel (20), said insert (100) including a plurality of ribs (115) extending substantially axially along the outside surface (111) of said central core (110), the outside diameter of the insert (100) over said ribs (115) being nearly identical to the inside diameter of said expulsion channel (20), such that the insert (100) is held in said channel (20) by jamming via said ribs (115).

11. A device according to claim 9 or 10, in which the insert (100) includes at least three ribs (115) which are regularly distributed around the central core (110) so that the central core (110) is centered in the middle of said expulsion channel (20).

12. A device according to any one of claims 9 to 11, in which the insert (100) includes four identical ribs (115) that are diametrically opposite in pairs around said central core (110).

13. A device according to any one of claims 9 to 12, in which said at least one rib (115) extends axially over the central core (110) parallel to the longitudinal axis of the expulsion channel (20).

14. A device according to any one of claims 9 to 12, in which said at least one rib (115) extends axially over the central core (110) in twisting manner about the longitudinal axis of the expulsion channel (20) such that the insert (100) establishes a turbulent air flow in the metering chamber (10).

15. A device according to any one of claims 3 to 14, in which the cross-section of the downstream end (110a) of said central core (110) of the insert (100) in the flow direction of the air flow is greater than the cross-section of the upstream end (110b) of said central core (110) such that the insert (100) establishes a flow of air that penetrates into the metering chamber (10) on a conical path.

16. A device according to any preceding claim, in which said flow means (100) include at least one groove or rib extending helically along the inside wall of the expulsion channel (20).

17. A device according to any preceding claim, in which said metering chamber (10) is defined in part by a metering element (200) disposed in fixed manner in the expulsion channel (20), said metering element (200) being disposed downstream from said flow means (100) in the flow direction of said air flow.

18. A device according to claim 17, in which said metering element (200) is disposed remotely from the walls of the expulsion channel (20) in such a manner that the air flow flows over the entire periphery of said metering element (200).

19. A device according to claim 17 or 18, in which said metering element (200) is terminated in a point (220) at its downstream end in the air flow direction.

20. A device according to any one of claims 17 to 19, in which said metering element (200) is fixed to said flow means (100).

21. A device according to claim 20, in which said flow means (100) are implemented in the form of an insert (100) fixed to a fixing member (300), said fixing member (300) being fixed axially in line (20a) with the expulsion channel (20) such that the insert (100) projects into said expulsion channel (20) away from the walls of said channel (20), the air flow flowing over the entire periphery of said insert (100).

22. A device according to claim 21, in which said insert (100) is of rectangular cross-section and said metering element (200) is of circular cross-section.

23. A device according to claim 21 or 22, in which said metering element (200), said insert (100), and said fixing member (300) are made as a single piece.

24. A device according to any one of claims 17 to 23, in which said metering element (200) includes a zone (210), such as a cup, defining the volume of the metering chamber (10).

25. A device according to any preceding claim, in which said air flow is a flow of compressed air created in the dispenser by a prestressed pump device which is triggerable during actuation of the dispenser.

## Patentansprüche

1. Vorrichtung zum Erleichtern des Entleerens einer Dosierkammer (10) einer Abgabevorrichtung für ein pulverförmiges Produkt, wobei die Abgabevorrichtung einen Ausstoßkanal (20) umfaßt, der sich durch die Dosierkammer (10) hindurch erstreckt, sowie Einrichtungen zum Zuführen eines Luftstroms, der dazu bestimmt ist, bei einer Betätigung der Abgabevorrichtung durch den Ausstoßkanal (20) hindurch zu strömen, um die Dosierkammer (10) zu entleeren, wobei die Vorrichtung Strömungsmittel (100) umfaßt, die geeignet sind, den Luftstrom derart zu konfgurieren, daß er die Dosierkammer (10) vollständig entleert, dadurch gekennzeichnet, daß die Strömungsmittel (100) im Ausstoßkanal (20) außerhalb und in Strömungsrichtung der Luftströmung stromaufwärts von der Dosierkammer (10) angeordnet sind.

2. Vorrichtung nach Anspruch 1, bei der die Dosierkammer (10) wenigstens einen Teil mit einem querverlaufenden Querschnitt aufweist, der größer ist, als der quer verlaufende Querschnitt des Ausstoßkanals (20), wodurch wenigstens eine Randzone (11) gebildet wird, wobei die Strömungsmittel (20) in der Lage sind, wenigstens einen Teil des Luftstroms in die wenigstens eine Randzone (11) der Dosierkammer (10) derart zu lenken, daß die Dosierkammer (10) vollständig entleert wird, worin die wenigstens eine Randzone (11) mit eingeschlossen ist.

3. Vorrichtung nach Anspruch 1 oder 2, bei der die Strömungsmittel (100) einen Einsatz (100) umfassen der fest im Ausstoßkanal angeordnet ist.

4. Vorrichtung nach Anspruch 3, bei der der Einsatz (100) einen zentralen, vollen Kern (110) umfaßt, wobei die Luftströmung längs der äußeren Oberfläche (111) dieses zentralen Kerns (110) strömt.

5. Vorrichtung nach Anspruch 3, bei der der Einsatz (100) einen zentralen Kern (110) umfaßt, der eine axiale Öffnung (112) längs seiner zentralen Längsachse aufweist, wobei ein Teil der Luftströmung durch diese axiale Öffnung (112) und der Rest längs der Außenoberfläche (111) des zentralen Kerns (110) strömen.

6. Vorrichtung nach einem der Ansprüche 3 oder 5, bei der der Einsatz (100) derart dimensioniert ist, daß er durch Einklemmen fest im Ausstoßkanal (20) gehalten wird.

7. Vorrichtung nach einem der Ansprüche 3 bis 6, bei der der Ausstoßkanal (20) einen kreisförmigen oder länglichen quer verlaufenden Querschnitt und der Einsatz (100) einen polygonalen quer verlaufenden Querschnitt aufweisen.

8. Vorrichtung nach einem der Ansprüche 3 bis 6, bei der der Ausstoßkanal (20) einen polygonalen Querschnitt und der Einsatz (100) einen kreisförmigen oder länglichen quer verlaufenden Querschnitt aufweisen.

9. Vorrichtung nach einem der Ansprüche 3 bis 8, bei der der Einsatz (100) weiterhin wenigstens eine Rippe (115) umfaßt, die sich ungefähr axial längs der Außenoberfläche (111) des zentralen Kerns (110) erstreckt.

10. Vorrichtung nach Anspruch 9, bei der der Ausstoßkanal (20) einen im wesentlichen kreisförmigen quer verlaufenden Querschnitt und der Kern (110) des Einsatzes (100) einen im wesentlichen kreisförmigen quer verlaufenden Querschnitt aufweisen, der kleiner ist als der des Ausstoßkanals (20), wobei der Einsatz (100) mehrere Rippen (115) aufweist, die sich ungefähr axial längs der äußeren Oberfläche (111) des zentralen Kerns (110) erstrecken, wobei der Außendurchmesser des Einsatzes (100) im Bereich der Rippen (115) ungefähr identisch mit dem Innendurchmesser des Ausstoßkanals (20) derart ist, daß der Einsatz (100) durch Einklemmen im Bereich dieser Rippen (115) im Kanal (20) gehalten wird.

11. Vorrichtung nach Anspruch 9 oder 10, bei der der Einsatz (100) wenigstens drei Rippen (115) umfaßt, die regelmäßig verteilt um den zentralen Kern (110) herum derart angeordnet sind, daß der zentrale Kern (110) in der Mitte des Ausstoßkanals (20) zentriert ist.

12. Vorrichtung nach einem der Ansprüche 9 bis 11, bei der der Einsatz (100) vier identische Rippen (115) umfaßt, die einander paarweise gegenüberliegend um den zentralen Kern (110) herum angeordnet sind.

13. Vorrichtung nach einem der Ansprüche 9 bis 12, bei der sich die wenigstens eine Rippe (115) axial auf dem zentralen Kern (110) parallel zur Längsachse des Ausstoßkanals (20) erstreckt.

14. Vorrichtung nach einem der Ansprüche 9 bis 12, bei der sich die wenigstens eine Rippe (115) in axialer Richtung über den zentralen Kern (110) bezüglich der Längsachse des Ausstoßkanals (20) schraubenförmig derart erstreckt, daß der Einsatz (100) in der Dosierkammer (10) eine verwirbelte Luftströmung erzeugt.

15. Vorrichtung nach einem der Ansprüche 3 bis 14, bei der der quer verlaufende Querschnitt des in Strömungsrichtung der Luftströmung stromabwärts gelegenen Endes (110a) des zentralen Kerns (110) des Einsatzes (100) größer ist als der quer verlaufende Querschnitt des stromaufwärts liegenden Endes (110b) des zentralen Kerns (110) derart, daß der Einsatz (100) eine Luftströmung erzeugt, die in die Dosierkammer (10) längs einer konischen Trajektorie eindringt.

16. Vorrichtung nach einem der vorhergehenden Ansprüche, bei der die Strömungsmittel (100) wenigstens eine Rille oder Rippe umfassen, die sich schraubenförmig auf der Innenwand des Ausstoßkanals (20) erstreckt.

17. Vorrichtung nach einem der vorhergehenden Ansprüche, bei der die Dosierkammer (10) teilweise von einem Dosierelement (200) begrenzt wird, das fest im Ausstoßkanal (20) angeordnet ist, wobei das Dosierelement (200) in Strömungsrichtung der Luftströmung stromabwärts von den Strömungsmitteln (100) angeordnet ist.

18. Vorrichtung nach Anspruch 17, bei der das Dosierelement (200) von den Wänden des Ausstoßkanals (20) derart entfernt angeordnet ist, daß die Luftströmung über den gesamten Umfang des Dosierelementes (200) strömt.

19. Vorrichtung nach Anspruch 17 oder 18, bei der das Dosierelement (200) an seinem in Richtung der Luftströmung abwärts gelegenen Ende in einer Spitze (220) endet.

20. Vorrichtung nach einem der Ansprüche 17 bis 19, bei der das Dosierelement (200) an den Strömungsmitteln (100) befestigt ist.

21. Vorrichtung nach Anspruch 20, bei der die Strömungsmittel (100) in der Form eines Einsatzes (100) realisiert sind, der an einem Befestigungsorgan (300) befestigt ist, wobei das Befestigungsorgan (300) in einer axialen Verlängerung (20a) des Ausstoßkanals (20) derart befestigt ist, daß der Einsatz (100) in den Ausstoßkanal (20) im Abstand von den Wänden dieses Kanals (20) hinein vorspringt, so daß die Luftströmung über die gesamte Umfangsoberfläche des Einsatzes (100) strömt.

22. Vorrichtung nach Anspruch 21, bei der der Einsatz (100) einen rechtwinkeligen quer verlaufenden Querschnitt und das Dosierelement (200) einen kreisförmigen quer verlaufenden Querschnitt aufweisen.

23. Vorrichtung nach Anspruch 21 oder 22, bei der das Dosierelement (200), der Einsatz (100) und das Befestigungsorgan (300) einstückig ausgeführt sind.

24. Vorrichtung nach einem der Ansprüche 17 bis 23, bei der das Dosierelement (200) eine Zone (210) z.B. in Form einer Schale umfaßt, die das Volumen der Dosierkammer (10) definiert.

25. Vorrichtung nach einem der vorhergehenden Ansprüche, bei der die Luftströmung ein Strom komprimierter Luft ist, der in der Abgabevorrichtung von einer vorgespannten und bei der Betätigung der Abgabevorrichtung auslösbaren Pumpenvorrichtung erzeugt wird.
